# EUROPEAN PATENT SPECIFICATION

(11) **EP 3 248 121 B1**
(45) Date of publication and mention of the grant of the patent: **03.03.2021**
(21) Application number: 16702823.2
(22) Date of filing: 06.01.2016
(51) Int. Cl.: G16H 40/63, G16H 40/67, G16H 20/40, G16H 10/60, A61M 1/16

(54) **REMOTE MONITORING INTERFACE DEVICE AND MOBILE APPLICATION FOR MEDICAL DEVICES**
FERNÜBERWACHUNGSSCHNITTSTELLENVORRICHTUNG UND MOBILE ANWENDUNG FÜR MEDIZINISCHE VORRICHTUNGEN
DISPOSITIF D'INTERFACE DE SURVEILLANCE À DISTANCE ET APPLICATION MOBILE POUR DISPOSITIFS MÉDICAUX

(30) Priority: 20.01.2015 US 201514600107
(43) Date of publication of application: 29.11.2017
(73) Proprietor: Fresenius Medical Care Holdings, Inc., Waltham, MA 02451 (US)
(72) Inventor: TANENBAUM, Lee, Walnut Creek, CA 94598 (US); WANG, Fei, Concord, CA 94521 (US); MOK, Aleo, Orinda, CA 94563 (US); DOYLE, Matthew, Concord, CA 94520 (US)
(74) Representative: Freischem & Partner Patentanwälte mbB
(86) International application number: PCT/US2016/012263
(87) International publication number: WO 2016/118318

(56) References cited:
- US-A1- 2013 274 642
- US-A1- 2013 297 330
- US-A1- 2014 184 422

## Description

### TECHNICAL FIELD

This patent application is related to processing devices and interfaces in the medical device area.

### BACKGROUND OF THE INVENTION

Hemodialysis is a process which employs a machine that includes a dialyzer to aid patients whose renal function has deteriorated to the point where their body cannot adequately rid itself of toxins. The dialyzer may include a semi-permeable membrane, the membrane serving to divide the dialyzer into two chambers. Blood is pumped through one chamber and a dialysis solution through the second. As the blood flows by the dialysis fluid, impurities, such as urea and creatinine, diffuse through the semi-permeable membrane into the dialysis solution. The electrolyte concentration of the dialysis fluid may be set so as to maintain electrolytic balance within the patient. Other purification techniques and processes may additionally be used. Hemodialysis may be generally referred to herein as "dialysis," although it is noted that other types of dialysis exist, such a peritoneal dialysis, and it is noted that the system described herein may be used in connection with any appropriate dialysis system or similar treatment system.

Since dialysis involves removing blood from and returning blood to a patient, performing a dialysis procedure carries a degree of risk. Dialysis treatment requires monitoring of several patient vital signs and dialysis parameters during the dialysis process in order to optimize the overall efficacy of the dialysis procedure, to assess the condition of a fistula (the access to the patient's blood) and to determine the actual purification achieved. Some examples of parameters monitored and analyzed by a dialysis machine or equipment include the blood access flow rate or the rate at which blood flows out of the patient to the dialyzer, a critical parameter, and the ratio Kt/V to measure dialysis efficiency, where K is the clearance or dialysance (both terms representing the purification efficiency of the dialyzer), t is treatment time and V is the patient's total water value.

A processing device coupled to the dialysis machine may be used to manage and oversee the functions of the dialysis process and to, for example, monitor, analyze and interpret patient vital signs and dialysis parameters during a dialysis procedure. The processing device may include a display that displays information concerning the dialysis procedure and include an interface that enables configuration and control of the dialysis machine. A health care practitioner (HCP) such as a nurse or a patient care technician may oversee the dialysis treatment sessions. Data provided by the dialysis machine and the processing device may aid the health care practitioner in performing his or her duties.

For various descriptions of dialysis systems and components, reference is made, for example, to US Patent No. 8,110,104 B2 to Crnkovich et al., entitled "Dialysis Systems and Related Components," and US Patent No. 6,775,577 B2 to Crnkovich et al., entitled "Method and System for Controlling a Medical Device". For a description of a sensor system that may be used in connection with monitoring and issuing alerts during a dialysis procedure, reference is made, for example, to US Patent No. 7,973,667 B2 to Crnkovich et al., entitled "Wetness Sensor". For various descriptions of interfaces for dialysis systems, reference is made, for example, to US Patent No. 8,323,503 B2 to Levin et al., entitled "User Interface Processing Device" and US Pub. No. 2007/0112603 A1 to Kauthen et al., entitled "Digital Data Entry Methods and Devices".

During a dialysis treatment, when thresholds or limits at the dialysis machine are reached and/or violated, such as in connection with patient variables including blood pressure, heart rate, monitored parameter levels, etc., alarms and/or other actions may be triggered at the dialysis machine. The result of this may cause the treatment to be suspended and/or prematurely stopped at the dialysis machine which may cause complications and problems during the dialysis treatment. Accordingly, it would be desirable to provide a system that facilitates the mitigation and/or avoidance of such problems or complications during a dialysis treatment.

US 2013/0297330 A1, US 2013/0274642 A1 and US 2014/0184422 A1 are prior art.

### SUMMARY OF THE INVENTION

According to the system described herein, a method for remotely monitoring a medical treatment includes executing a remote monitoring application on a remote interface device. The method further includes receiving data, using the remote monitoring application, concerning the medical treatment being performed with a medical device. A first limit is set using the remote monitoring application, wherein the first limit is a limit for a treatment parameter measured during the medical treatment that is reached before a second limit set at the medical device for the treatment parameter is reached. An indicator is indicated on the remote interface device when the first limit is reached. In response to the indicator on the remote interface device, a remedial action is initiated in connection with the treatment parameter before the second limit is reached at the medical device. The medical treatment is a dialysis treatment, and the medical device is a dialysis machine. The second limit, when reached, causes an alarm or a suspension of the medical treatment at the medical device. The remote interface device may be portable or wearable by a user, in particular, the remote interface device may be a smart phone or a tablet device and/or the remote interface device may be worn by the user. The first limit may be set on the remote monitoring application by a user according to a preference of the user. The data concerning the medical treatment may be streamed from the medical device directly to the remote interface device and/or the data concerning the medical treatment may be first stored on a server, and the remote interface device accesses the data via the server. The data may be encrypted. Executing the remote monitoring application on the remote interface device may include requiring authorized access credentials from a user to log into the remote monitoring application.

According further to the system described herein, a non-transitory computer-readable medium stores software for remotely monitoring a medical treatment. The software includes executable code for a remote monitoring application installed on a remote interface device. Executable code is provided that receives data, using the remote monitoring application, concerning the medical treatment being performed with a medical device. Executable code is provided that enables setting a first limit on the remote monitoring application, wherein the first limit is a limit for a treatment parameter measured during the medical treatment that is reached before a second limit set at the medical device for the treatment parameter is reached. Executable code is provided that indicates with an indicator on the remote interface device when the first limit is reached. Executable code is provided that, in response to the indicator on the remote interface device, enables initiating of a remedial action in connection with the treatment parameter before the second limit is reached at the medical device. The medical treatment is a dialysis treatment, and the medical device is a dialysis machine. The second limit, when reached, causes an alarm or a suspension of the medical treatment at the medical device. The remote interface device may be portable or wearable by a user, in particular, the remote interface device may be a smart phone or a tablet device and/or the remote interface device may be worn by the user. The first limit may be set on the remote monitoring application by a user according to a preference of the user. The data concerning the medical treatment may be streamed from the medical device directly to the remote interface device and/or the data concerning the medical treatment may first be stored on a server, and wherein the remote interface device accesses the data via the server. The data may be encrypted. The executable code for the remote monitoring application on the remote interface device may include executable code that requires authorized access credentials from a user to log into the remote monitoring application.

According further to the system described herein, a system for remote monitoring of a medical treatment includes a medical device and a remote interface device. A non-transitory computer-readable medium stores software for remotely monitoring the medical treatment. The software includes executable code for a remote monitoring application installed on a remote interface device. Executable code is provided that receives data, using the remote monitoring application, concerning the medical treatment being performed with the medical device. Executable code is provided that enables setting a first limit on the remote monitoring application, wherein the first limit is a limit for a treatment parameter measured during the medical treatment that is reached before a second limit set at the medical device for the treatment parameter is reached. Executable code is provided that indicates with an indicator on the remote interface device when the first limit is reached. Executable code is provided that, in response to the indicator on the remote interface device, enables initiating of a remedial action in connection with the treatment parameter before the second limit is reached at the medical device. The medical treatment is a dialysis treatment, and the medical device is a dialysis machine. The second limit, when reached, causes an alarm or a suspension of the medical treatment at the medical device. The remote interface device may be portable or wearable by a user, in particular, the remote interface device may be a smart phone or a tablet device and/or the remote interface device may be worn by the user. The first limit may be set on the remote monitoring application by a user according to a preference of the user. The data concerning the medical treatment may be streamed from the medical device directly to the remote interface device and/or the data concerning the medical treatment may first be stored on a server, and wherein the remote interface device accesses the data via the server. The data may be encrypted. The executable code for the remote monitoring application on the remote interface device may include executable code that requires authorized access credentials from a user to log into the remote monitoring application.

### BRIEF DESCRIPTION OF THE DRAWINGS

Embodiments of the system described herein are explained with reference to the several figures of the drawings, which are briefly described as follows.
FIG. 1 is a schematic illustration of an example of a patient care environment in which a patient seated in a chair receives medical treatment from a dialysis machine and which may be used in connection with an embodiment of the system described herein.
FIG. 2 is a schematic illustration of another example of a patient care environment that may be used in connection with an embodiment of the system described herein.
FIG. 3 is schematic illustration of an example implementation of the dialysis machine that may be used in connection with an embodiment of the system described herein.
FIG. 4 is a schematic illustration of a more detailed implementation of the dialysis machine that may be used in connection with an embodiment of the system described herein.
FIG. 5 is a schematic illustration showing example information that may be displayed on the display of the dialysis machine in connection with an embodiment of the system described herein.
FIG. 6 is a schematic illustration according to an embodiment of the system described herein showing the HCP using an interface device in connection with the monitoring and/or control of a dialysis machine in connection with a dialysis treatment being performed in the patient care environment.
FIG. 7 is a schematic illustration of a remote interface device on which an application may be executed that enables remotely receiving data from a dialysis machine according to an embodiment of the system described herein.
FIG. 8 is a schematic illustration of another embodiment for a remote interface device that may be configured as wearable device in accordance with an embodiment of the system described herein.
FIG. 9 is a flow diagram showing processing, using a remote monitoring application, for remote monitoring of a medical treatment being performed with a medical device according to an embodiment of the system described herein.

### DETAILED DESCRIPTION OF VARIOUS EMBODIMENTS

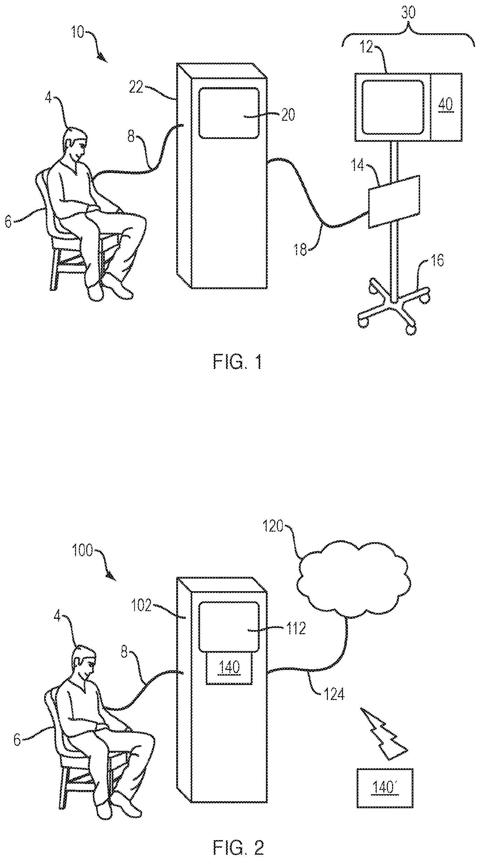

FIG. 1 is a schematic illustration of an example of a patient care environment 10 in which a patient 4 seated in a chair 6 receives medical treatment from a treatment station 22 and which may be used in connection with an embodiment of the system described herein. The medical treatment is dialysis. The treatment station 22 is a dialysis treatment station or dialysis machine. A tube or blood line 8 transports blood from the patient 4 to the dialysis machine 22 and back again to the patient 4 after processing and treatment in the dialysis machine 22. The dialysis machine 22 with display 20 may be connected via cabling 18 to a controller device 30 that may include a processor 14 which controls a display 12. In various embodiments, the display 20 may display information corresponding to a dialysis treatment being performed by the dialysis machine 22. The display 12 may be mounted on a movable stand 16 of the controller device 30. The display 12 permits a health care practitioner (HCP), such as a nurse, a patient care technician (PCT), or even a patient, to interface with the display 12 to, for example, to monitor and/or control the dialysis machine 22 and/or to enter patient or other data, for example. In various embodiments, the display 12 may include touch screen display capability as well as be implemented in connection with non-contact or gesture based detection and control capability, among other possible interfacing features.

According to various embodiments of the system described herein, a transceiver device 40 may be coupled to the controller device 30 that may be used to control the dialysis machine 22, as further discussed in detail elsewhere herein. In an embodiment, the signal transceiver device 40 may include a sensor to detect control input by a user or HCP operating the dialysis machine 22, and may include touch based control input, such as a via touch screen, and/or non-contact input, such as by a gesture detection and recognition device. Further, the signal transceiver device 40 may also include wireless communication capability in connection with wireless coupling to one or more wireless interface devices that may be used by an HCP to monitor and/or control a dialysis treatment being performed by the dialysis machine 22, as further discussed elsewhere herein. Various embodiments for the one or more wireless interface devices and for the actions and functions of the signal transceiver device 40 in connection with control of the dialysis machine 22 are further discussed in detail elsewhere herein. It is noted that where reference is made herein to wireless coupling or communication, the system described herein may be used with any appropriate wireless communication technology, including, for example, IEEE 802.11b/g, 802.11b/g/n and/or Bluetooth, having appropriate security and encryption standards and used in conjunction with appropriate wireless networks, having hardware and software components, that support such wireless communication technologies.

FIG. 2 is a schematic illustration of another example of a patient care environment 100 that may be used in connection with an embodiment of the system described herein. In the patient care environment 100, the patient 4 is seated in the chair 6 and receives medical treatment from a treatment station, such as a dialysis machine 102. The tube or blood line 8 is used for transporting blood from the patient 4 to the dialysis machine 102 and back again to the patient 4 after processing and treatment of the blood in the dialysis machine 102. The dialysis machine 102 may be configured to communicate with an external network 120, such as a local-area network or the Internet, via a wired or wireless connection 124. The network 120 may include one or more databases or other stores of information that securely contain medical information that may be accessed in connection with operation of the system described herein. It is noted that the system described herein may be used in connection with dialysis products produced by Fresenius Medical Care North America of Waltham, Massachusetts, including, for example, one or more Fresenius hemodialysis systems (e.g., a 2008T system).

In an embodiment, the dialysis machine 102 may include a display 112. The dialysis machine 102 may centralize and consolidate dialysis functions and data entry functions in a single device 102, without, e.g., the use of a separate, external display (e.g., display 12 of FIG. 1) or a separate, external processor (e.g., processor 14) with associated equipment (e.g., movable stand 16). In an embodiment, the dialysis machine 102 may include one or more processors 114, like the processor 14, that may be used in connection with interfacing with, and control of, the dialysis machine 102, for example, by an HCP during a dialysis treatment. Consolidation of functions in a single dialysis machine 102 may advantageously reduce the amount of external cabling (e.g., cabling 18) to the device 102. The dialysis machine 102 may further reduce the amount of space needed for dialysis treatment and present less crowding of the patient care environment 100. An HCP may be able to focus solely on the dialysis machine 102, or the display 112 of the dialysis machine 102, without the HCP's attention being diverted to, e.g., another external display. The dialysis machine 102 may reduce power consumption and cost as compared to other, non-centralized implementations.

In an embodiment, a signal transceiver device 140 may be coupled to the dialysis machine 102. The signal transceiver device 140 may have similar features and functionality as the signal transceiver device 40 discussed elsewhere herein. As noted in connection with the signal transceiver device 40 of FIG. 1, and as discussed in detail elsewhere herein, the signal transceiver device 140 may also include wireless channel components that may be used in connection with receiving external or remote signals that may be used to control the dialysis machine 102 and/or may be transmit signals in connection with operation of the dialysis machine 102. In another embodiment, a signal transceiver device 140 may be located separately or remotely from the dialysis machine 102 and coupled wirelessly thereto. Further, in an embodiment, the signal transceiver device 140 may also be wirelessly coupled to the network 120. Accordingly, in various embodiments, functions of the signal transceiver device 140 may include control of and/or information exchange with the dialysis machine 102 via direct communication therewith and/or the signal transceiver device 140 may interface with the dialysis machine 102 via the network 120.

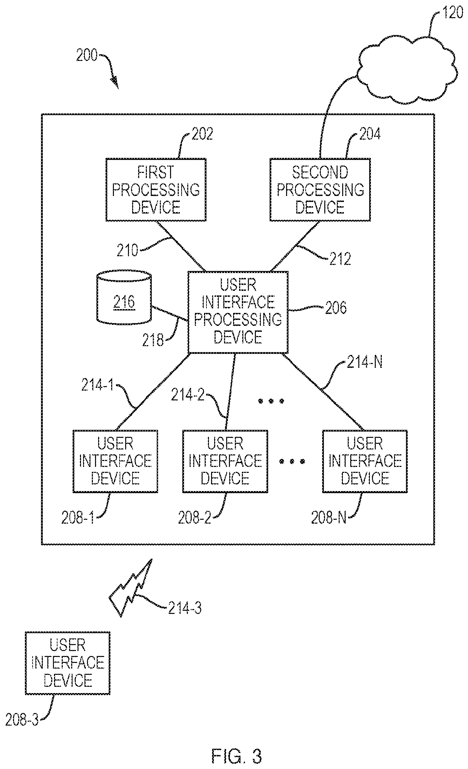

FIG. 3 is schematic illustration of an example implementation 200 of the dialysis machine 102 according to an embodiment of the system described herein. A user interface processing device (UIP) 206 may be configured to share user interface resources, i.e., user interface devices 208-1, 208-2, 208-3 ... , 208-N, between a first processing device 202 and a second processing device 204. Both the first and the second processing devices 202, 204 may be connected to the UIP 206 via respective connections 210, 212, while the user interface devices 208-1, 208-2, 208-3 ... , 208-N are connected to the UIP 206 via connections 214-1, 214-2, 214-3 ... , 214-N. Although one UIP 206 is shown in FIG. 3, several user interface processing devices may be used to implement the functionality of the UIP 206. The UIP 206 is connected to memory 216 via a connection 218. Other memory (not shown) may be connected to, and, used by, e.g., the first processing device 202 and/or the second processing device 204. The second processing device 204 of the device 200 may be configured to communicate with the external network 120, such as a local-area network or the Internet, via a wired or wireless connection 124 (and, e.g., via a network interface (not shown)). In other implementations, other processing devices such as the UIP 206 or the first processing device 202 may communicate with an external network such as the external network 120.

The user interface devices 208-1, 208-2, 208-3 ... , 208-N may include any of a variety of user interface devices known in the art, such as an alphanumeric keyboard or a keypad, a pointing device (e.g., a touchpad, a mouse, or a trackball) and/or a display with a touch screen, such a tablet, mobile phone and/or other portable processing and display device. Accordingly, in various implementations, one or more of the user interface devices 208-1, 208-2, 208-3... , 208-N may be directly disposed on or coupled to the device 200 and/or may be located external to the device 200 and coupled via wired and/or wireless connections to the device 200. Specifically, for example, user interface device 208-3 is shown remotely located and wirelessly coupled, via wireless connection 214-3, to the device 200. Various embodiments for a user interface device, like that of the user interface device 208-3, that may be used to wirelessly monitor and/or control the dialysis machine 102 are further discussed in detail elsewhere herein and may include use of one or more portable processing or interfacing devices, such as a tablet or mobile phone, that are wirelessly coupled to the dialysis machine 102 to provide and/or receive wireless signals to control and/or monitor the display 112 and/or other information generated in connection with the dialysis machine 102.

As described herein, the UIP 206 may be configured to share the user interface devices 208-1, 208-2, 208-3 ... , 208-N between the first processing device 202 and the second processing device 204. The UIP 206 may switch focus from the first processing device 202 to the second processing device 204. The UIP 206 may likewise switch focus from the second processing device 204 to the first processing device 202. Specifically, a processing device, such as the first or the second processing device 202, 204 of FIG. 3, may be said to have focus when the processing device has control of, and/or is controlled by, one or more user interface devices connected to, or communicating with, the processing device (e.g., via one or more user interface processing devices). That is, in this example, when a processing device has focus, a user interface device connected to, or communicating with, the processing device (e.g., via one or more user interface processing devices) will generally affect operation of the processing device, and thereby the dialysis machine 102. User interactions with a user interface device will likewise generally affect operation of the processing device in this instance. Likewise, in this example, when a processing device has focus, the processing device may control a user interface device (such as a video display) connected to, or communicating with, the processing device (e.g., via one or more user interface processing devices).

When a processing device, such as the first or the second processing device 202, 204 of FIG. 3, does not have focus, then, for example, the processing device may not have control of and/or be controlled by one or more user interface devices connected to, or communicating with, the processing device (e.g., via one or more user interface processing devices). Rather, another processing device may have been given focus. One or more user interface processing devices such as the UIP 206 may send protocol data to the processing device, even when the processing device does not presently have focus, so that the processing device may be configured to maintain connections with one or more user interface devices. That is, from the perspective of the processing device, even when the processing device does not have focus, the processing device may have a connection maintained with a user interface device that the processing device does not control and/or that is not controlled by the processing device when the processing device does not have focus. The UIP 206 may therefore send protocol data related to the one or more user interface devices to the first and the second processing devices 202, 204, irrespective of which processing device 202, 204 has focus.

When a processing device (such as the first processing device 202 or the second processing device 204) has focus, one or more user interface processing devices (such as the UIP 206) may manage communications between one or more user interface devices (such as the user interface devices 208-1, 208-2, 208-3 ... , 208-N) and the processing device. The UIP 206 may, when the processing device has focus, permit the user interface devices 208-1, 208-2, 208-3 ... , 208-N to affect operation of the processing device. The UIP 206 may switch between modes. The modes may be exclusive of one another and may include a mode in which the first processing device 202 has focus, and a mode in which a second processing device 204 has focus.

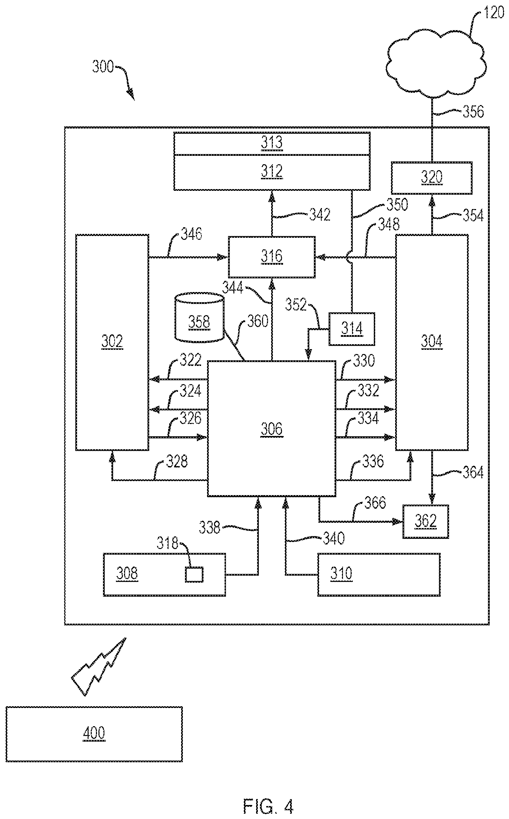

FIG. 4 is a schematic illustration of a more detailed implementation 300 of the dialysis machine 102 according to an embodiment of the system described herein. A UIP 306 is configured to share user interface resources, such as a keyboard 308 and a pointing device 310 (such as a touchpad) between a first processing device 302 and a second processing device 304. Further user interface components, according to the system described herein, may include a display 312, like the display 112 discussed herein, that may be coupled to or otherwise integrated with an input receiver/controller 313. In various embodiments, the display 312 may also further include touch screen input control features, such as touch screen controller 314. In other embodiments, the system described herein may operate in connection with use of a remote interface device 400 that may be wirelessly coupled to the dialysis machine 102 for control and/or monitoring of the display 112/312 thereof, as further discussed in detail herein. It is noted that the first processing device 302 may be a functional dialysis processing device (FHP) 302 that may be configured to monitor dialysis functions of the device 300. The second processing device 304 may be a microprocessor, such as a standard personal computer (PC) processor, embedded within the device 300, and may be referred to as an embedded processing device (EP) 304. The FHP 302 is connected to the UIP 306 via connections 322, 324, 326, 328, and the EP 304 is connected to the UIP 306 via connections 330, 332, 334, 336.

The keyboard 308 is connected to the UIP 306 via connection 338. The pointing device 310 is connected to the UIP 306 via connection 340. The display 312 may be connected to a digital video switch 316 via connection 342, which is in turn connected to the UIP 306, the FHP 302, and the EP 304 via respective connections 344, 346, 348. A touch screen controller 314 may be connected to the display 312 via connection 350, and to the UIP 306 via connection 352. Although one UIP 306 is shown in FIG. 4, several user interface processing devices may be used to implement the functionality of the UIP 306. The UIP 306 is connected to memory 358 via a connection 360. Other memory (not shown) may be connected to, and, used by, e.g., the FHP 302 and/or the EP 304. The EP 304, for example, may utilize a flash memory rather than a conventional hard drive. The device 300 also includes an audio device 362. The audio device 362 is connected to the EP 304 via connection 364 and the UIP 306 via connection 366. FIG. 4 is intended to show functional connections between devices of the device 300, so more or fewer connections may be used than are shown in FIG. 4.

As described above, the UIP 306 may switch focus from the FHP 302 to the EP 304. The UIP 306 may likewise switch focus from the EP 304 to the FHP 302. When the FHP 302 has focus, one or more of the keyboard 308, the pointing device 310, the display 312 with a touch screen will generally affect operation of the FHP 302. When the EP 304 has focus, the keyboard 308, the pointing device 310, the display 312, and/or the remote interface device 400 may generally affect operation of the EP 304. User interactions with the devices 308, 310, 312, 400 will likewise generally affect operation of whichever processing device (the FHP 302 or the EP 304) has focus. The processing device that has focus (the FHP 302 or the EP 304) may control, e.g., the display 312 in certain circumstances.

In various implementations, one or more of the user interface devices may be located external to the device 300. In this example implementation, when the EP 304 has focus, the FHP 302 does not have focus, and the FHP 302 may not have control of and/or be controlled by the devices 308, 310, 312, 400. When the FHP 302 has focus, the EP 304 does not have focus, and the EP 304 may not have control of and/or be controlled by the devices 308, 310, 312, 400. The UIP 306 may send protocol data relating to the devices 308, 310, 312 to the EP 304 and the FHP 302, even when one of these devices does not have focus, so that the EP 304 and the FHP 302 may maintain connections with the devices 308, 310, 312. That is, from the perspective of the processing device (EP 304 or FHP 302) that does not have focus, a connection at least appears to be maintained with the devices 308, 310, 312, 400, even though these devices 308, 310, 312, 400 are not controlled by, and do not control, the processing device that does not have focus. The UIP 306 may therefore send protocol data related to the devices 308, 310, 312, 400 to the FHP 302 and the EP 304, irrespective of which processing device 302, 304 has focus. The UIP 306 may switch between modes. The modes may be exclusive of one another and may include a mode in which the first processing device 302 has focus, and a mode in which the second processing device 304 has focus.

The interface device 400 may include one or more advisory indictors, such as lights, that may communicate a successful pairing of the interface device with the signal transceiver device 140 and/or the dialysis machine 102. The advisory indicators may further communicate other information, such as low battery and/or other information concerning the communication pathway between the interface device 400 and the dialysis machine 102.

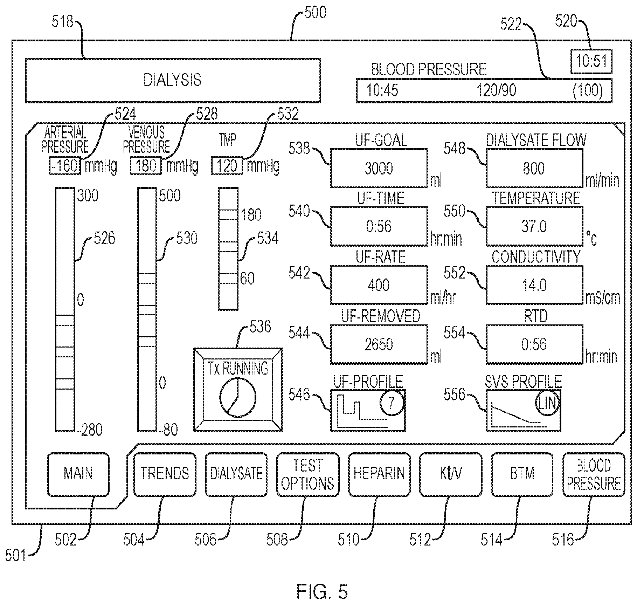

FIG. 5 is a schematic illustration showing an embodiment of information 500 that may be displayed on the display 112 of the dialysis machine 102 in connection with an embodiment of the system described herein. The illustrated embodiment of the information 500 is presented by way of example only, and other information, particularly other operational functions and features for controlling and/or monitoring a dialysis treatment, may be displayed and/or controlled in accordance with the system described herein. In various embodiments, the system described herein may be used in connection with generation and/or display of information corresponding to a medical device, medical treatment and/or patient related data. In the illustrated embodiment, the information 500 may include a treatment screen on the display 112 of the dialysis machine 102 that incorporates methods and systems for monitoring and/or controlling functions of the dialysis machine 102 like that discussed elsewhere herein. It is noted that other appropriate treatment information may be provided and used in connection with monitoring and/or controlling a dialysis machine and/or other medical device in connection with the system described herein.

Screen access buttons 502 (main access), 504 (trends), 506 (dialysate), 508 (test options), 510 (heparin), 512 (Kt/V), 514 (BTM), and 516 (blood pressure) may be used to access the various treatment screens in a manner that may be similar to that accessed at the display 112. For example, as illustrated, the main access button 502 has been activated revealing a main treatment access screen 501 that may be displayed on the display 112 of the dialysis machine. It is noted that, in other embodiments, different and/or summarized versions of the information displayed on the display 112 of the dialysis machine 102 may be displayed on the interface device 400. A different treatment access screen may be displayed, for example, by pressing the different screen access buttons. The main treatment access screen 501 provides a general overview of the status of the current treatment. Other treatment screens may offer a more in-depth view of specific aspects of the current treatment, though some treatment screens may have some of the same information displayed as found on other treatment screens.

A status box 518 appears at the top left corner of the treatment screen being displayed in the information 500. During normal operation it displays the operation mode of the machine, which in this case is "Dialysis." During alarm situations, a warning message may be displayed in the status box 518. The message displayed in the status box 518 may also prompt the operator for a specific action in situations when the treatment parameters are being set. During normal treatment, a box 520 displays the current time and the box 522 displays the time of the last blood pressure reading and the patient's blood pressure and pulse rate at that time. Arterial pressure in mmHg is displayed numerically in a meter box 524, and graphically in a bar graph 526. Similarly, venous pressure in mmHg is displayed numerically in a meter box 528 and graphically in a bar graph 530, and transmembrane pressure (TMP) in mmHg is displayed numerically in a meter box 532 and graphically in a bar graph 534.

A Tx clock button 536 may be activated start, or to pause or suspend, the treatment. The Tx clock button 536 controls multiple functions of the hemodialysis machine when it is activated. A UF-goal button 538 displays the desired ultrafiltration (UF) in milliliters to be removed during the dialysis treatment. This is typically the difference between the patient's pre and dry weight plus saline or fluid intake during treatment. The UF-time button 540 acts as a countdown timer displaying the remaining time in hours and minutes that ultrafiltration will be performed. The timer stops during a blood alarm or whenever the UF pump is stopped. During treatment, A UF-rate button 542 displays the current rate of ultrafiltration in milliliters per hour. The rate ultrafiltration occurs is determined by the values entered in a UF-goal button 538 and a UF-time button 540 and the profile selected with a UF-profile button 546. A UF-removed button 544 keeps a running total in milliliters of the fluid drawn from the patient through ultrafiltration. When the value displayed in the UF-Removed button 544 is equal to the value entered in the UF-goal button 538, an alarm sounds and the message, "UF GOAL REACHED" is displayed in the status box 518. A UF-profile button 546 when touched brings up the UF Profile selection screen. Once a profile is selected, and the operator pushes the main access button 502, the profile selected is displayed in the UF-profile button 546.

A dialysate flow button 548 displays the current dialysate flow rate in milliliters per minute. A temperature button 550 displays the current temperature in degrees centigrade of the dialysate. Pressing the temperature button 550 allows the operator to set the desired temperature, and thereafter the actual temperature is displayed. If the temperature varies too far from the set point, an alarm sounds, a warning message is displayed in the status box 518, and the dialysate goes into bypass. A conductivity button 552 displays the current conductivity in millisiemens per centimeter of the dialysate. An RTD (Remaining Time of Dialysis) button 554 acts as a countdown timer displaying the amount of treatment time remaining. At the end of treatment (RTD=0:00) an alarm sounds and the message "RTD ZERO" is displayed in the status box 518. An SVS profile button 556 when touched brings up the Sodium Variation System (SVS) profile selection screen. Once a profile is selected, and the operator pushes the main access button 502, the profile selected is displayed in the SVS profile button 556.

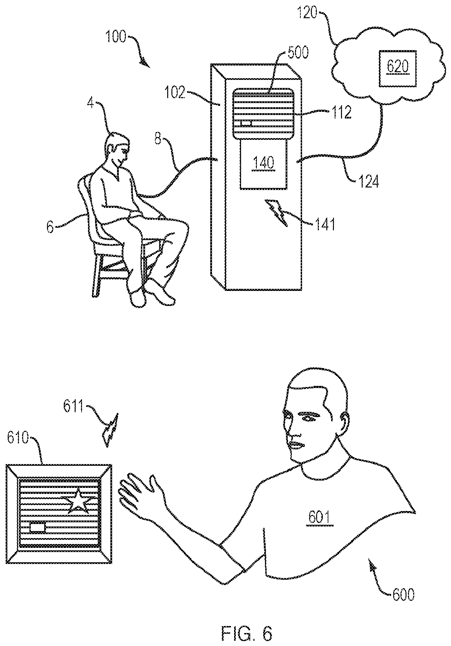

FIG. 6 is a schematic illustration 600 according to an embodiment of the system described herein showing an HCP 601 using an interface device 610 in connection with the monitoring and/or control of a dialysis machine in connection with a dialysis treatment being performed in the patient care environment 100. In this embodiment, the HCP 601 is shown in proximity to the interface device 610 which may be an implementation of the interface device 400. The interface device 610 enables the HCP 601 to monitor and/or control the dialysis machine 102 during the dialysis treatment in a non-contact manner without requiring the HCP to touch and/or otherwise contact the dialysis machine 102 or component thereof or be in direct line of sight with the dialysis machine.

Using the interface device 610, the HCP 601 may navigate through screens being displayed on the display of the interface device 610 and monitor portion of the information 500 being displayed on the dialysis machine 102. In other embodiments, the interface device 610 may be used to select or activate portions of the information 500 being displayed. In an embodiment, the interface device 610 may be wirelessly coupled to the dialysis machine 102, and specifically to the display 112 of the dialysis machine 102, in which content for display on the display 112 of the dialysis machine 102 is also, or instead, displayed on the display of the interface device 610. The wireless channel for wireless signal transmission and reception to/from the interface device 610 is shown schematically as wireless channel 611 of the interface device 610. It is noted that the information displayed on the interface device 610 may not necessarily be identical to the information displayed on the display 112, but instead may be summarized or condensed version thereof, for example.

Further shown schematically is wireless channel 141 that may be a wireless channel for transmission of signals from wireless channel components of the signal transceiver device. For example, over the wireless channel pairing signals and/or acknowledge signal may be communicated among the signal transceiver device 140 and the interface device 610, as well as information corresponding to the display of information, and control of the information, of the dialysis machine 102 in accordance with the embodiments discussed herein. It is noted that the wireless communication channel between the interface device 610 and the signal transceiver device 140 may include suitable relay components for relaying the signal via the network 120, that may be an internal network and/or an external network, such as the Internet, in connection with operation of the system for the described embodiment(s). Using the interface device 610, the HCP 601 may control the dialysis machine during the dialysis treatment without contacting the display 112 of the dialysis machine 102 and/or without necessarily contacting the interface device 610. In this way, the HCP 601 does not need to re-glove each time an action needs to be taken with respect to the dialysis machine 102, such as modifying a parameter thereof during the dialysis treatment. In various embodiments, the information used by the interface device 610 may be generated from patient data streamed directly to the interface device 610 and/or accessed from one or more servers 620 via the network 120. The patient data may be streamed and/or stored on the one or more servers 620 according to appropriate security and encryption protocols and standards and securely accessed by the interface device 610 according to the required input of authorized access credentials from the user when logging into a remote monitoring application installed and executing on the interface device 610, as further discussed elsewhere herein.

In various embodiments, the interface device 610 may be used to control functionality of the treatment screen being displayed as information 500 on the interface device 610. Accordingly, the mechanism of control of the treatment screen may deviate from control of the treatment screen that is being displayed on the display 112 of the dialysis machine 102. For example, whereas the treatment screen displayed on the screen of the interface device 610 may be controlled, for example, by the command-based recognition that may be used to iterate through and/or highlight different buttons of the information 500 for the treatment screen that is being displayed on the display 112. As discussed elsewhere herein, in other embodiments, the information 500 being displayed on the interface device 610 may present a treatment screen that is somewhat different from the treatment screen presented on the display 112 of the dialysis machine 102 in a manner that facilitates that command-based recognition control enabled by the interface device 610.

Dialysis machines may include sensors or other components for monitoring patient parameters and other treatment-related information during a dialysis treatment. The information may be transmitted from the dialysis machine to a storage device or monitoring device. For a discussion of detecting medical parameters and/or conditions of a patient receiving medical treatment, such as by sensing blood pressure, heart rate, weight, glucose level, hemoglobin level and/or blood potassium level, for example, and transmitting information regarding the parameters to a digital data processing system disposed remotely from the medical treatment apparatus, reference is made to US Pub. No. 2010/0137693 A1 to Porras et al, entitled "Methods and Systems for Patient Care". Reference is also made to US Pub. No. 2013/0018355 A1 to Brand et al., entitled "Method and Device for Remote Monitoring and Control of Medical Fluid Management Devices," and to US Pub. No. 2013/0211206 A1 to Sands et al., entitled "Patient Treatment and Monitoring Systems and Methods with Cause Inferencing".

According to the system described herein, a remote monitoring mobile application may be installed and executed on a remote interface device (e.g., smartphone, tablet, computer, etc.) that may have features like the one or more of the interface devices discussed elsewhere herein. By streaming the treatment data and/or a log or summary of the treatment data, attending HCPs may have continual access to the dialysis machine and patient data at the treatment center. In various embodiments, the data may be streamed directly to the remote interface device and/or first transmitted to a server that is accessible by the remote interface device. The operator is able to execute, or securely log into, the application on the remote interface device to access dialysis machine data, patient data and/or other treatment-related information during the treatment. The HCP, through the application, is then able to monitor the data of the treatment at the treatment center. The remote monitoring mobile application may be software downloaded from a network, such as the Internet and/or an intranet, and/or otherwise installed on the remote interface device. The remote monitoring mobile application may include executable code that is executed to perform functions and having capabilities to interact with components of the remote interfacing device to receive data concerning a medical treatment being performed a different location by a medical device and to process and analyze that data to determine whether one or more soft limits, set at the remote monitoring mobile application, has been reached.

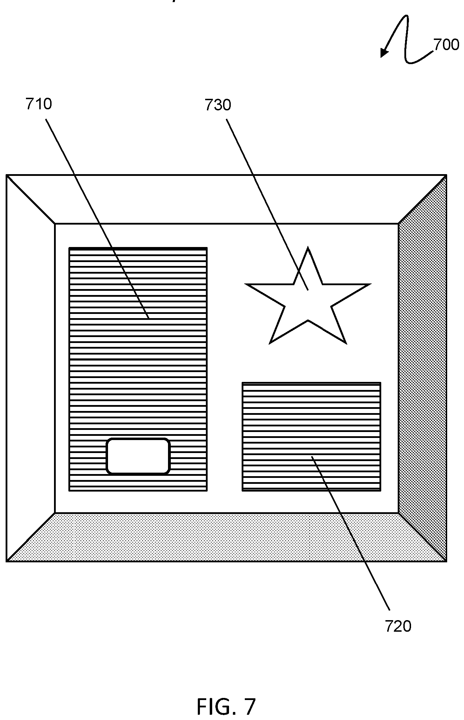

FIG. 7 is a schematic illustration of a remote interface device 700 on which an application may execute that enables remotely receiving data in connection with a dialysis treatment at a dialysis machine according to an embodiment of the system described herein. In various embodiments, the remote interface device 700 may have similar features as one or more of the interface devices discussed elsewhere herein, such as the remote interface device 610, and including a transceiver to receive wireless signals and/or to output wireless signals. In an embodiment, patient data and/or other treatment-related data may be transmitted to, and/or made accessible to, the remote interface device. In an embodiment, the data may be streamed to an accessible server and/or directly to the remote interface device 700. In an embodiment, the remote monitoring application executing on the remote interface device 700 may display information 710 concerning the dialysis machine and/or treatment that may be remotely viewed by an HCP using the remote interface device 700. In various embodiments, the information 710 received by the remote interface device 700 may be generated from data streamed and/or accessed by the remote interface device 700 that is transmitted and/or stored according to appropriate security and encryption protocols and standards in compliance with applicable laws and regulations concerning sensitive patient data and/or other medical information, as further discussed elsewhere herein.

In an embodiment, the application executing on the remote interface device 700 may have one or more input features 720 to enable setting "soft" thresholds or limits on patient-related variables, such as blood pressure, heart rate or other monitored blood parameter levels, and/or other treatment-related information. The soft limits are limits set only on the application, and thereby on the remote interface device 700, and not on the dialysis machine, such as the dialysis machine 102. When a soft limit input on the application via the input features 720 is reached and/or otherwise violated, an indicator 730 is triggered on the remote interface device 700 that may inform the HCP that the soft limit has been reached or violated. The indicator 730 may be a warning symbol and/or other indicator disposed or displayed on the remote interface device 700 and includes information as to which soft limit set by the HCP has been reached.

The soft limits may be configurable and customized by the HCP on the remote interface device 700 according to the specifications of the HCP, and customized to trigger the indicator to the HCP before other hard limits at dialysis machine are reached and/or violated. The hard limits at the dialysis machine are limits set according to explicit regulations or guidelines, whereas the soft limits may be customized limits set individually by the HCP, for example, according to preference of the HCP. The indicator 730 includes timing information that indicates how long before a hard limit at the dialysis machine is reached whereupon an alarm and/or other treatment-disruptive action would occur at the dialysis machine, as well as other appropriate or desirable information concerning the soft limit. Accordingly, the soft limits set by the HCP may be individually configured by the HCP based on location of the HCP, based on specific conditions of the patient known to the HCP, and/or based other variables that are designed to thereby give sufficient time for the HCP to address the problem during the treatment before the hard limit is reached at the dialysis machine.

Through these soft limits, the HCP may be notified about significant changes or rates of change in treatment parameters before the changes exceed the dialysis machine's set alarm limits. By addressing the issue before the dialysis machine alarm is triggered, and/or by alerting the HCP personally even when the HCP is remote from the dialysis machine, the application on the remote interface device 700 may facilitate the taking of preemptive action by the HCP and/or other practitioner before the occurrence of the treatment-halting alarms triggered at the dialysis machine, and particularly in the situation where the HCP is initially remote from the dialysis machine. In this way, the hemodialysis treatment may progress more smoothly and may thereby enable preemptive avoidance of unnecessary complications during the treatment.

The information and/or data concerning the treatment parameters received by the remote monitoring application at the remote interface device 700 may be streamed directly to the remote interface device 700 from one or more medical devices and/or may be accessed by the remote interface device 700 from one or more servers, e.g. server 620. In various embodiments, particularly for security purposes and appropriate protection of sensitive patient information under applicable laws and regulations, the application on the remote interface device 700 may require secure log in by a user requiring user input of credentials to facilitate authorized access by appropriate HCPs to the patient information using the application. The credentials may include secure access credentials, including usernames, passwords, personal identification numbers (PINs), biometric credentials, appropriate near field communication (NFC) credentials, and/or other appropriate access credentials. The data streamed to the remote interface device 700 and/or stored on the one or more servers, e.g., server 620, may be encrypted using appropriate encryption protocols and techniques, and subsequently decrypted using appropriate decryption protocols and techniques in connection with use thereof by the application on the remote interface device 700.

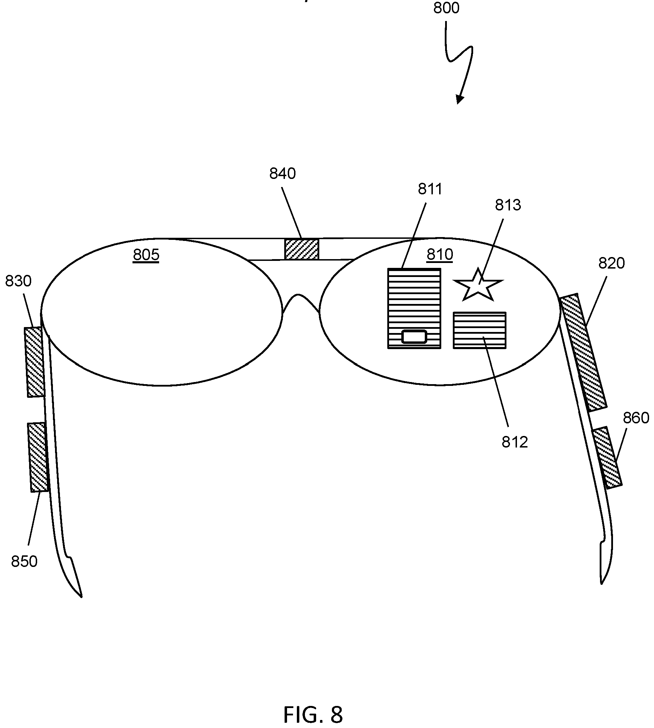

FIG. 8 is a schematic illustration of another embodiment for a remote interface device 800, that may have features like the remote interface devices discussed elsewhere herein, but configured as wearable device in accordance with an embodiment of the system described herein. The wearable device may be a head-mounted display that may include smart glasses, such as a Google Glass device, and/or other appropriate type of head-mounted display. Other implementations of wearable devices may be appropriately used in connection with the system described herein, such as a smart watch implementation. The remote interface device 800 may be worn by a user, such as an HCP, in connection with remotely monitoring and/or controlling a dialysis machine or component during a dialysis treatment, as further discussed in detail elsewhere herein. In various other embodiments, the remote interface device 800 may be a display that is coupled to a different portable processing device, such as a smart phone or tablet, and display information being transmitted from portable processing device.

The remote interface device 800 may include two screens or sides 805, 810 that may be used and function independently of each other. For example, in an embodiment, the side 805 may be clear to enable a user to perform duties remote from the dialysis machine and unobstructed by any visual display during use of the interface device 800, and the side 810 may display information 811, 812, 813 used in connection with monitoring and/or controlling the dialysis machine 102 and/or component thereof. In other embodiments, different types of information sent to, or generated by, the remote interface device 800 may be displayed on either of the sides 805, 810 and, when not in use, both of the sides may be transparent, for example.

The interface device 800 may receive data and information wirelessly transmitted from the dialysis machine 102 and/or otherwise made accessible to the interface device 800, such as via a mobile-accessible server. In an embodiment, information displayed on one or more of the sides 805, 810 may indicate a successful pairing of the interface device 800 with the dialysis machine 102 in connection with streaming of data from the dialysis machine 102 to the interface device 800. In other embodiments, the side 805 may also display other information such as identification information to help ensure the HCP is matching the patient being treated with the proper dialysis machine information being viewed on the interface device 800.

In an embodiment, the information 811, 812, 813 displayed on the side 810 may be information similar to that of the information discussed in connection with the remote interface device 700. Specifically, the information may include monitoring information 811 of the dialysis machine or treatment, input information 812 in connection with soft limits entered by the HCP and an indicator 813 when soft limits are reached and/or otherwise violated. According to the system described herein, in a clinical or dialysis setting, while an HCP is performing other duties, including duties for other patients, remote from the dialysis machine 102 and dialysis treatment being monitored by the HCP, using the interface device 800, the HCP may remotely monitor, and/or control, the dialysis treatment and be informed when soft limits set by the HCP for the treatment have been reached or violated without having to physically contact the dialysis machine 102 and/or even without having to be physically present at the dialysis machine 102.

In an embodiment, the remote interface device 800 may include a transceiver device 820 that receives and/or transmits signals according to the functionality discussed herein. The transceiver device 820 may include one or more processors to process the signals in connection with the display of the information 811, 812, 813 and in connection with transmission of instructions for remotely controlling the dialysis machine 102. The transceiver device 820 may further include a memory, and/or other non-transitory computer-readable media, to store data in connection with the information transmitted and/or received by the interface device 800 and in connection with the execution of software or other executable code in connection with the operations of the interface device 800, specifically, execution of the remote monitoring application discussed elsewhere herein.

For example, the interface device 800 may include a command recognition device 830 that recognizes and interprets commands in connection with operation of the interface device 800, specifically in connection with selection, control and activation of elements on the screens 805, 810 of the interface device 800. In various embodiments, the commands may be gestures recognized and used by a gesture-recognition module of the command recognition device 830 in connection with the operation of the interface device 800 may include, for example, hand gestures, head gestures and/or eye gestures of the user. In other embodiments, the command recognition device 830 may include a voice recognition module that enables voice-based control of the interface device 800. The command recognition capability thereby enables hands-free, non-contact operation of the interface device and remote control of the dialysis machine according to the system described herein.

In other embodiments, the interface device 800 may further include a camera 840 that may be used in connection with capturing images that may be displayed and/or used by the interface device 800. In various embodiments, the camera 840 may also include video capabilities. It is noted that although the devices 820, 830 and 840 are shown as separate devices, in other embodiments, the functionalities of these devices may be incorporate into one integral device disposed on the interface device 800. Further, the interface device 800 may include a power source 850, such as a battery.

The interface device 800 may further include an audio input/output device 860 that may include a speaker component and a microphone component. The audio device 860 may enable the user to hear audible signals that may be transmitted by the dialysis machine 102. In other embodiments the dialysis machine 102 may also include a speaker and/or a microphone component, such that an intercom-type verbal exchange may be enabled between the HCP wearing the interface device 800 and a patient at the dialysis machine 102. For example, the HCP may then communicate with the patient, and/or with other HCPs or nurses, when the indicator 813 indicates to the HCP that one or more soft limits set at the application on the remote interface device 800 have been reached and/or violated.

In various embodiments, the audio device 860 may also operate in connection with voice command recognition capability of the command recognition device 830, as further discussed elsewhere herein. It is also noted that the processing of the interface device 800 may enable the interface device to recognize a verbal communication from the patient that is then converted into text and displayed on one or more of the screens 805, 810. In connection therewith, the processing of the interface device 800 may enable translation capabilities. For example, a patient may make a verbal communication at the dialysis machine 102 in one language (such as Spanish) and the verbal communication is transmitted as a signal to the interface device 800, where the verbal communication is converted into text and then translated into another language (such as English) via processing capabilities of the interface device 800 and the translated text displayed on one or more of the screens 805, 810 of the interface device 800.

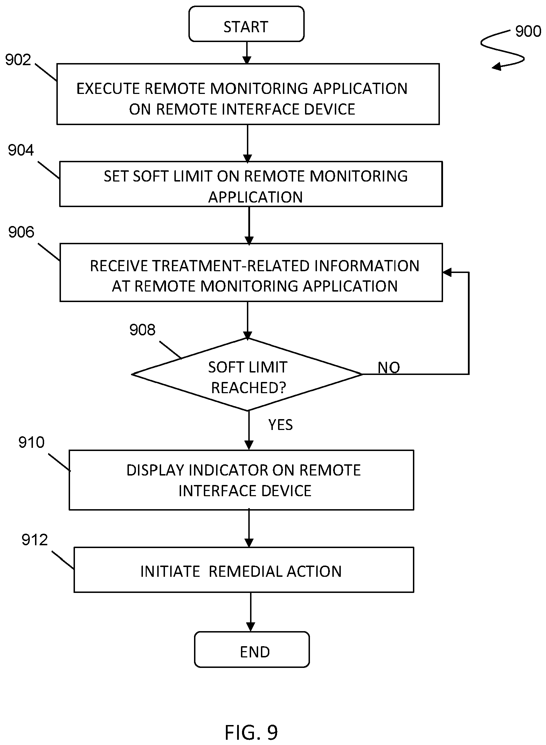

FIG. 9 is a flow diagram 900 showing processing, using a remote monitoring application, for remote monitoring of a medical treatment being performed with a medical device according to an embodiment of the system described herein. At a step 902, a user (e.g. HCP) executes a remote monitoring application installed on a remote interface device, like that discussed elsewhere herein, that may be actuated, carried and/or worn by the user. After the step 902, at a step 904, the user sets or activates soft limits, through the remote monitoring application on the remote interface device, for treatment parameters of a dialysis treatment that may be occurring at a location remote from the location of the user. The soft limits are limits set to be reached prior to hard limits set at the dialysis machine that cause alarms and/or other treatment-disruptive actions at the dialysis machine, such as suspension of the dialysis treatment. After the step 904, at a step 906, the remote monitoring application on the remote interface device receives data concerning the dialysis treatment being performed. In various embodiments, the data may be received at the remote monitoring application directly from the dialysis machine, such as via streamed treatment data, and/or the remote monitoring application may receive the data by accessing a server to which the data has been initially transmitted and/or stored. As further discussed elsewhere herein, in various embodiments the data may be streamed and/or stored on the server using appropriate security and encryption protocols and standards to ensure access to sensitive patient data only by authorized HCPs that have securely logged into the remote monitoring application using appropriate access credentials.

After the step 906, at a test step 908, it is determined whether a soft limit set by the user has been reached. If not, then processing proceeds back to the step 906. If, at the test step 908, it is determined that a soft limit has been reached, then processing proceeds to a step 910 where an indicator is displayed on the remote interface device indicating that the soft limit has been reached. The indicator may indicate that the soft limit has been reached using a warning symbol and may also provide information as to which soft limit has been reached. After the step 910, processing proceeds to a step 912 where a remedial action is initiated to attempt to avoid the reaching of a hard limit for the treatment parameter at the dialysis machine that would cause an alarm and/or other treatment-disruptive action at the dialysis machine. In an embodiment, in some circumstances, the HCP may initiate the remedial action using the remote interface device where appropriate and without necessarily requiring the presence of the HCP at the dialysis machine. In other circumstances, the presence of the HCP may be required at the dialysis machine and/or the patient in order for the HCP to take the remedial action and the HCP may be warned to proceed immediately to the dialysis machine and patient. After the step 912, processing is complete for the iteration of the processing being described. Other iterations of the processing may then be further performed in connection with the system described herein.

It is noted that the system described herein is discussed principally in connection with the use of dialysis machines and treatments. In other embodiments, the system described herein may also be used in connection with other medical devices where remote monitoring of such devices is desirable and may be appropriately performed. It is also noted that the system described herein may be used in connection and conjunction with the features and functions of systems for controlling or monitoring medical devices like that described in US Pub. No. 2014/0267003 A1 to Wang et al., entitled "Wireless Controller to Navigate and Activate Screens on a Medical Device," US Pub. No. 2014/0266983 A1 to Christensen, entitled "Wearable Interface for Remote Monitoring and Control of a Medical Device," and US Patent App. No. 14/200,156 to Merics et al., filed March 7, 2014, entitled "E-Field Sensing of Non-Contact Gesture Input for Controlling a Medical Device".

Various embodiments discussed herein may be combined with each other in appropriate combinations in connection with the system described herein. Additionally, in some instances, the order of steps in the flow diagrams, flowcharts and/or described flow processing may be modified, where appropriate. Further, various aspects of the system described herein may be implemented using software, hardware, a combination of software and hardware and/or other computer-implemented modules or devices having the described features and performing the described functions. The system may further include a display and/or other computer components for providing a suitable interface with a user and/or with other computers.

Software implementations of the system described herein may include executable code that is stored in a computer-readable medium and executed by one or more processors. The computer-readable medium may include volatile memory and/or non-volatile memory, and may include, for example, a computer hard drive, ROM, RAM, flash memory, portable computer storage media such as a CD-ROM, a DVD-ROM, an SD card, a flash drive or other drive with, for example, a universal serial bus (USB) interface, and/or any other appropriate tangible or non-transitory computer-readable medium or computer memory on which executable code may be stored and executed by a processor. The system described herein may be used in connection with any appropriate operating system.

Other embodiments of the invention will be apparent to those skilled in the art from a consideration of the specification or practice of the invention disclosed herein. It is intended that the specification and examples be considered as exemplary only, with the scope of the invention being indicated by the following claims.

## Claims

1. A method for remotely monitoring a medical treatment, comprising:
executing a remote monitoring application on a remote interface device (700, 800);
receiving data, using the remote monitoring application, concerning the medical treatment being performed with a medical device (102);
setting a soft limit on a patient-related variable using the remote monitoring application, wherein the soft limit is a limit for a treatment parameter measured during the medical treatment that is reached before reaching a hard limit set at the medical device (102) for the treatment parameter, and wherein the soft limit is only settable on the remote interface device (700, 800);
indicating with an indicator (730, 830) on the remote interface device (700, 800) when the soft limit is reached, wherein the indicator (730, 830) includes information as to which soft limit set has been reached;
and
in response to the indicator (730, 830) on the remote interface device (700, 800), initiating a remedial action in connection with the treatment parameter before the hard limit is reached at the medical device (102), wherein the hard limit, when reached, causes an alarm or a suspension of the medical treatment at the medical device (102),
**characterized by**
setting the hard limit according to explicit regulations or guidelines at the medical device (102) for the treatment parameter, and by
indicating with timing information included by the indicator (730, 830) how long before the hard limit at the medical device (102) is reached, wherein the medical treatment is a dialysis treatment, and wherein the medical device (102) is a dialysis machine.

2. The method according to claim 1, wherein the remote interface device (700, 800) is portable or wearable by a user (601).

3. The method according to claim 2, wherein the remote interface device (700, 800) is a smart phone or a tablet device.

4. The method according to claim 2, wherein the remote interface device (700, 800) is worn by the user (601).

5. The method according to claim 1, wherein the soft limit is set on the remote monitoring application by a user (601) according to a preference of the user (601).

6. The method according to claim 1, wherein the data concerning the medical treatment is streamed from the medical device (102) directly to the remote interface device (700, 800).

7. The method according to claim 1, wherein the data concerning the medical treatment is first stored on a server (620), and wherein the remote interface device (700, 800) accesses the data via the server (620).

8. The method according to claim 1, wherein the data concerning the medical treatment is streamed from the medical device (102) or stored on a server (620), and wherein the data is encrypted.

9. The method according to claim 1, wherein executing the remote monitoring application on the remote interface device (700, 800) includes requiring authorized access credentials from a user (601) to log into the remote monitoring application.

10. A non-transitory computer-readable medium storing software for remotely monitoring a medical treatment, the software comprising:
executable code that implements the method of one of the preceding claims, to be executed by a remote interface device (700, 800) receiving data, using a remote monitoring application installed on the remote interface device (700, 800), concerning the medical treatment performed with a medical device (102), wherein the remote interface device (700, 800) has an indicator (730, 830), wherein the medical treatment is a dialysis treatment, and wherein the medical device is a dialysis machine.

11. A system for remote monitoring of a medical treatment, comprising:
a medical device (102);
a remote interface device (700, 800);
a non-transitory computer-readable medium storing software for remotely monitoring the medical treatment, the software being executed by at least one processor of the remote interface device (700, 800) and including:
executable code for a remote monitoring application installed on the remote interface device (700, 800);
executable code that receives data, using the remote monitoring application, concerning the medical treatment being performed with the medical device (102);
executable code that enables setting a soft limit on a patient-related variable using the remote monitoring application, wherein the soft limit is a limit for a treatment parameter measured during the medical treatment that is reached before reaching a hard limit set at the medical device (102) for the treatment parameter, and wherein the soft limit is only settable on the remote interface device (700, 800);
executable code that indicates with an indicator (730, 830) on the remote interface device (700, 800) when the soft limit is reached, wherein the indicator (730, 830) includes information as to which soft limit set has been reached; and
executable code that, in response to the indicator (730, 830) on the remote interface device (700, 800), enables initiating of a remedial action in connection with the treatment parameter before the hard limit is reached at the medical device (102), wherein the hard limit, when reached, causes an alarm or a suspension of the medical treatment at the medical device (102),
**characterized by**
executable code that sets the hard limit according to explicit regulations or guidelines at the medical device (102) for the treatment parameter, and by
executable code that indicates with timing information included by the indicator (730, 830) how long before the hard limit at the medical device (102) is reached, wherein the medical treatment is a dialysis treatment and wherein the medical device (102) is a dialysis machine (102).

12. The system according to claim 11, wherein the soft limit is set on the remote monitoring application by a user (601) according to a preference of the user (601).

## Patentansprüche

1. Verfahren zur Fernüberwachung einer medizinischen Behandlung, umfassend:
Ausführen einer Fernüberwachungsanwendung auf einer Fernschnittstellen-Vorrichtung (700, 800);
Empfangen von Daten, unter Verwendung der Fernüberwachungsanwendung, in Bezug auf die mit einer medizinischen Vorrichtung (102) durchgeführte medizinische Behandlung;
Festlegen einer weichen Grenze für eine patientenbezogene Variable unter Verwendung der Fernüberwachungsanwendung, wobei die weiche Grenze ein Grenzwert für einen während der medizinischen Behandlung gemessenen Behandlungsparameter ist, welcher erreicht wird, bevor eine an der medizinischen Vorrichtung (102) für den Behandlungsparameter festgelegte harte Grenze erreicht wird, und wobei die weiche Grenze nur an der Fernschnittstellen-Vorrichtung (700, 800) festgelegt werden kann;
Anzeigen mit einer Anzeige (730, 830) an der Fernschnittstellen-Vorrichtung (700, 800), wenn die weiche Grenze erreicht ist, wobei die Anzeige (730, 830) Informationen darüber umfasst, welche festgelegte weiche Grenze erreicht wurde;
und
als Reaktion auf die Anzeige (730, 830) an der Fernschnittstellen-Vorrichtung (730, 830) Einleiten einer Abhilfemaßnahme in Verbindung mit dem Behandlungsparameter, bevor die harte Grenze an der medizinischen Vorrichtung (102) erreicht wurde, wobei die harte Grenze, wenn sie erreicht ist, einen Alarm oder eine Aussetzung der medizinischen Behandlung an der medizinischen Vorrichtung (102) bewirkt,
**gekennzeichnet durch**
Festlegen der harten Grenze gemäß ausdrücklichen Bestimmungen oder Richtlinien an der medizinischen Vorrichtung (102) für den Behandlungsparameter und durch
Anzeigen mit von der Anzeige (730, 830) umfassten Informationen zum zeitlichen Ablauf, wie lange es dauern wird, bis die harte Grenze an der medizinischen Vorrichtung (102) erreicht wird, wobei die medizinische Behandlung eine Dialysebehandlung ist und wobei die medizinische Vorrichtung (102) ein Dialysegerät ist.

2. Verfahren nach Anspruch 1, wobei die Fernschnittstellen-Vorrichtung (700, 800) tragbar ist oder von einem Benutzer (601) getragen werden kann.

3. Verfahren nach Anspruch 2, wobei die Fernschnittstellen-Vorrichtung (700, 800) ein Smartphone oder eine Tablet-Vorrichtung ist.

4. Verfahren nach Anspruch 2, wobei die Fernschnittstellen-Vorrichtung (700, 800) von dem Benutzer (601) getragen wird.

5. Verfahren nach Anspruch 1, wobei die weiche Grenze in der Fernüberwachungsanwendung durch einen Benutzer (601) gemäß einer Präferenz des Benutzers (601) festgelegt wird.

6. Verfahren nach Anspruch 1, wobei die Daten in Bezug auf die medizinische Behandlung von der medizinischen Vorrichtung (102) direkt an die Fernschnittstellen-Vorrichtung (700, 800) gestreamt werden.

7. Verfahren nach Anspruch 1, wobei die Daten in Bezug auf die medizinische Behandlung zunächst auf einem Server (620) gespeichert werden und wobei die Fernschnittstellen-Vorrichtung (700, 800) über den Server (620) auf die Daten zugreift.

8. Verfahren nach Anspruch 1, wobei die Daten in Bezug auf die medizinische Behandlung von der medizinischen Vorrichtung (102) gestreamt oder auf einem Server (620) gespeichert werden und wobei die Daten verschlüsselt sind.

9. Verfahren nach Anspruch 1, wobei die Ausführung der Fernüberwachungsanwendung auf der Fernschnittstellen-Vorrichtung (700, 800) die Anforderung von Zugangsberechtigungsdaten von einem Benutzer (601) umfasst, um sich in die Fernüberwachungsanwendung einzuloggen.

10. Nicht-transitorisches computerlesbares Medium, das Software zur Fernüberwachung einer medizinischen Behandlung speichert, wobei die Software Folgendes umfasst:
ausführbaren Code, welcher das Verfahren nach einem der vorangehenden Ansprüche implementiert, zur Ausführung durch eine Fernschnittstellen-Vorrichtung (700, 800), welche unter Verwendung einer auf der Fernschnittstellen-Vorrichtung (700, 800) installierten Fernüberwachungsanwendung Daten in Bezug auf die mit einer medizinischen Vorrichtung (102) durchgeführte medizinische Behandlung empfängt, wobei die Fernschnittstellen-Vorrichtung (700, 800) eine Anzeige (730, 830) aufweist, wobei die medizinische Behandlung eine Dialysebehandlung ist und wobei die medizinische Vorrichtung ein Dialysegerät ist.

11. System für die Fernüberwachung einer medizinischen Behandlung, umfassend:
eine medizinische Vorrichtung (102);
eine Fernschnittstellen-Vorrichtung (700, 800);
ein nicht-transitorisches computerlesbares Medium, welches Software zur Fernüberwachung der medizinischen Behandlung speichert, wobei die Software von mindestens einem Prozessor der Fernschnittstellen-Vorrichtung (700, 800) ausgeführt wird und Folgendes umfasst:
ausführbaren Code für eine auf der Fernschnittstellen-Vorrichtung (700, 800) installierte Fernüberwachungsanwendung;
ausführbaren Code, welcher unter Verwendung der Fernüberwachungsanwendung Daten in Bezug auf die mit der medizinischen Vorrichtung (102) durchgeführte medizinische Behandlung empfängt;
ausführbaren Code, welcher die Festlegung einer weichen Grenze für eine patientenbezogene Variable unter Verwendung der Fernüberwachungsanwendung ermöglicht, wobei die weiche Grenze ein Grenzwert für einen während der medizinischen Behandlung gemessenen Behandlungsparameter ist, welcher erreicht wird, bevor eine an der medizinischen Vorrichtung (102) für den Behandlungsparameter festgelegte harte Grenze erreicht wird, und wobei die weiche Grenze nur an der Fernschnittstellen-Vorrichtung (700, 800) festgelegt werden kann;
ausführbaren Code, welcher mit einer Anzeige (730, 830) an der Fernschnittstellen-Vorrichtung (700, 800) anzeigt, wenn die weiche Grenze erreicht ist, wobei die Anzeige (730, 830) Informationen darüber umfasst, welche festgelegte weiche Grenze erreicht wurde; und
ausführbaren Code, welcher als Reaktion auf die Anzeige (730, 830) an der Fernschnittstellen-Vorrichtung (730, 830) das Einleiten einer Abhilfemaßnahme in Verbindung mit dem Behandlungsparameter ermöglicht, bevor die harte Grenze an der medizinischen Vorrichtung (102) erreicht wurde, wobei die harte Grenze, wenn sie erreicht ist, einen Alarm oder eine Aussetzung der medizinischen Behandlung an der medizinischen Vorrichtung (102) bewirkt,
**gekennzeichnet durch**
ausführbaren Code, welcher die harte Grenze gemäß ausdrücklichen Bestimmungen oder Richtlinien an der medizinischen Vorrichtung (102) für den Behandlungsparameter festlegt, und durch
ausführbaren Code, welcher mit von der Anzeige (730, 830) umfassten Informationen zum zeitlichen Ablauf anzeigt, wie lange es dauern wird, bis die harte Grenze an der medizinischen Vorrichtung (102) erreicht wird, wobei die medizinische Behandlung eine Dialysebehandlung ist und wobei die medizinische Vorrichtung (102) ein Dialysegerät ist.

12. System nach Anspruch 11, wobei die weiche Grenze in der Fernüberwachungsanwendung durch einen Benutzer (601) gemäß einer Präferenz des Benutzers (601) festgelegt wird.

## Revendications

1. Méthode de surveillance à distance d'un traitement médical, comprenant les étapes suivantes:
l'exécution d'une application de surveillance à distance sur une interface distante (700, 800) ;
la réception de données concernant le traitement médical effectué à l'aide d'un dispositif médical (102) au moyen de l'application de surveillance à distance,
la fixation d'une limite souple pour une variable liée au patient à l'aide de l'application de surveillance à distance dans laquelle la limite souple est une limite d'un paramètre de traitement mesurée pendant le traitement médical qui est atteinte avant d'atteindre une limite stricte fixée dans le dispositif médical (102) pour le paramètre de traitement et dans laquelle la limite souple n'est réglable que sur le dispositif d'interface à distance (700, 800) ;
l'indication par un indicateur (730, 830) sur le dispositif d'interface à distance (700, 800) lorsque la limite souple est atteinte, dans lequel l'indicateur (730, 830) comprend des informations sur la limite souple qui a été atteinte; et
en réponse à l'indicateur (730, 830) sur l'interface à distance (700, 800), le déclenchement d'une action corrective en rapport avec le paramètre de traitement avant que la limite stricte ne soit atteinte au niveau du dispositif médical (102), dans lequel la limite stricte, lorsqu'elle est atteinte, provoque une alarme ou une suspension du traitement médical au niveau du dispositif médical (102),
**caractérisée par le fait que**
l'on fixe la limite stricte selon des réglementations ou des directives explicites au niveau du dispositif médical (102) pour le paramètre de traitement et que l'on indique avec des informations de temps incluses par l'indicateur (730, 830) combien de temps s'est écoulé avant que la limite stricte au niveau du dispositif médical (102) soit atteinte, dans laquelle le traitement médical est un traitement de dialyse, et dans laquelle le dispositif médical (102) est une machine de dialyse.

2. Méthode selon la revendication 1, dans laquelle le dispositif d'interface à distance (700, 800) est portable ou peut être porté par un utilisateur (601).

3. Méthode selon la revendication 2, dans laquelle le dispositif d'interface à distance (700, 800) est un téléphone intelligent ou un dispositif à tablette.

4. Méthode selon la revendication 2, dans laquelle le dispositif d'interface à distance (700, 800) est porté par l'utilisateur (601).

5. Méthode selon la revendication 1, dans laquelle la limite souple est fixée sur l'application de surveillance à distance par un utilisateur (601) selon une préférence de l'utilisateur (601).

6. Méthode selon la revendication 1, dans laquelle les données concernant le traitement médical sont transmises en continu du dispositif médical (102) directement au dispositif d'interface à distance (700, 800).

7. Méthode selon la revendication 1, dans laquelle les données concernant le traitement médical sont d'abord stockées sur un serveur (620), et dans laquelle le dispositif d'interface à distance (700, 800) accède aux données via le serveur (620).

8. Méthode selon la revendication 1, dans laquelle les données concernant le traitement médical sont diffusées en continu à partir de l'appareil médical (102) ou stockées sur un serveur (620), et dans laquelle les données sont cryptées.

9. Méthode selon la revendication 1, dans laquelle l'exécution de l'application de surveillance à distance sur le dispositif d'interface à distance (700, 800) comprend l'exigence d'une autorisation d'accès d'un utilisateur (601) pour se connecter à l'application de surveillance à distance.

10. Support non transitoire lisible par ordinateur stockant un logiciel pour la surveillance à distance d'un traitement médical, le logiciel comprenant :
un code exécutable qui met en oeuvre la méthode de l'une des revendications précédentes, destiné à être exécuté par un dispositif d'interface à distance (700, 800) recevant des données concernant le traitement médical effectué avec un dispositif médical (102), au moyen d'une application de surveillance à distance installée sur le dispositif d'interface à distance (700, 800), dans lequel le dispositif d'interface à distance (700, 800) a un indicateur (730, 830), dans lequel le traitement médical est un traitement de dialyse, et dans lequel le dispositif médical est une machine de dialyse.

11. Système de surveillance à distance d'un traitement médical, comprenant :
un dispositif médical (102) ;
un dispositif d'interface à distance (700, 800) ;
un support non transitoire lisible par ordinateur stockant un logiciel pour la surveillance à distance du traitement médical, le logiciel étant exécuté par au moins un processeur du dispositif d'interface à distance (700, 800) et comprenant :
du code exécutable pour une application de surveillance à distance installée sur le dispositif d'interface à distance (700, 800) ;
du code exécutable qui reçoit des données, en utilisant l'application de surveillance à distance concernant le traitement médical effectué avec le dispositif médical (102) ;
du code exécutable qui permet de fixer une limite souple sur une variable liée au patient en utilisant l'application de surveillance à distance, dans lequel la limite souple est une limite pour un paramètre de traitement mesuré pendant le traitement médical qui est atteinte avant d'atteindre une limite stricte fixée au niveau du dispositif médical (102) pour le paramètre de traitement, et dans lequel la limite souple est uniquement réglable sur le dispositif d'interface à distance (700, 800) ;
du code exécutable qui indique à l'aide d'un indicateur (730, 830) sur le dispositif d'interface à distance (700, 800) lorsque la limite souple est atteinte, l'indicateur (730, 830) comprenant des informations sur la limite souple qui a été atteinte ; et
du code exécutable qui, en réponse à l'indicateur (730, 830) sur le dispositif d'interface à distance (700, 800) permet de lancer une action corrective en rapport avec le paramètre de traitement avant que la limite stricte ne soit atteinte au niveau du dispositif médical (102), dans lequel la limite stricte, lorsqu'elle est atteinte, provoque une alarme ou une suspension du traitement médical au niveau du dispositif médical (102),
**caractérisé par le fait qu'**il comprend
du code exécutable qui fixe la limite stricte selon des réglementations ou des directives explicites au niveau du dispositif médical (102) pour le paramètre de traitement, et
du code exécutable qui indique avec des informations temporelles incluses par l'indicateur (730, 830) la durée de temps avant que la limite stricte au niveau du dispositif médical (102) soit atteinte, dans lequel le traitement médical est un traitement de dialyse et dans lequel le dispositif médical (102) est une machine de dialyse (102).

12. Système selon la revendication 11, dans lequel la limite souple est fixée sur l'application de surveillance à distance par un utilisateur (601) selon une préférence de l'utilisateur (601).
